# EUROPÄISCHE PATENTSCHRIFT

(11) **EP 0 834 506 B1**
(45) Veröffentlichungstag und Bekanntmachung des Hinweises auf die Patenterteilung: **14.01.2004**
(21) Anmeldenummer: 97116753.1
(22) Anmeldetag: 26.09.1997
(51) Int. Cl.: C07D 221/28

(54) **Verfahren zur Herstellung von (9alpha, 13alpha, 14alpha)-1-(3-Methoxymorphinan-17-yl)alkanonen**
Process for the preparation of (9alpha, 13alpha, 14alpha)-1-(3-Methoxymorphinan-17-yl)alkanones
Procédé pour la préparation des (9alpha, 13alpha, 14alpha)-1-(3-Méthoxymorphinan-17-yl)alkanones

(30) Priorität: 02.10.1996 EP 96115783
(43) Veröffentlichungstag der Anmeldung: 08.04.1998
(73) Patentinhaber: F. HOFFMANN-LA ROCHE AG, 4070 Basel (CH)
(72) Erfinder: Wehrli, Christof, 4108 Witterswil (CH)
(74) Vertreter: Kjellsaa-Berger, Hanny, Dr.

(56) Entgegenhaltungen:
- DE-A- 2 311 881
- FR-A- 1 584 396
- US-A- 4 857 648
- O. SCHNIDER ET AL.: HELVETICA CHIMICA ACTA, Bd. 33, Nr. 6, 1950, Seiten 1437-48, XP002050841
- M. KITAMURA ET AL.: TETRAHEDRON LETTERS, Bd. 28, Nr. 41, 1987, Seiten 4829-32, XP002050843

## Beschreibung

Die vorliegende Erfindung betrifft ein neues Verfahren zur Herstellung von (9α, 13α, 14α)-1-(3-Methoxymorphinan-17-yl)alkanonen der allgemeinen Formel 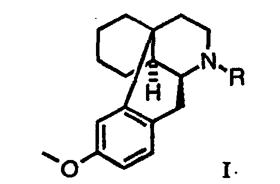 worin R C₂₋₄- Alkanoyl bedeutet,
durch Cyclisation eines (R)- oder (S)-1-[1-(4-Methoxybenzyl)-1,2,3,4,5,6,7,8-octahydro-isochinolin-2-yl]alkanons der allgemeinen Formel 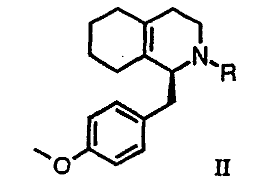 worin R die oben angegebene Bedeutung besitzt,
mit einer Alkylsulfonsäure.

Der in dieser Beschreibung verwendete Ausdruck " C₂₋₄- Alkanoyl" bedeutet sowohl geradkettige als auch verzweigte Reste einer aliphatischen Carbonsäure mit 2 bis 4 Kohlenstoffatomen, wie Acetyl, Propionyl, Butyryl, wobei Acetyl besonders bevorzugt ist.

Die (9α, 13α, 14α)-1-(3-Methoxymorphinan-17-yl)alkanone der obigen Formel I sind wichtige Ausgangsmaterialien zur Herstellung von Dextromethorphan der Formel

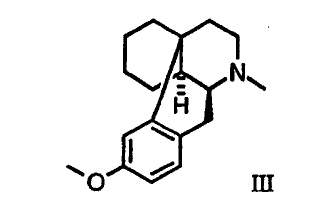

Sie können in an sich bekannter Weise, z.B. in Analogie zu den in der Deutschen Offenlegungsschrift 2 311 881 beschriebenen Methoden durch Abspaltung des Restes R und N-Methylierung, in Dextromethorphan übergeführt werden. Die Verwendung der (9α, 13α, 14α)-1-(3-Methoxymorphinan-17-yl)alkanone zur Herstellung von Dextromethorphan ist ebenfalls Gegenstand der vorliegenden Erfindung.

Es sind bereits verschiedene Verfahren zur Herstellung von Dextromethorphan bekannt. Ein solches Verfahren ist beispielsweise in Helv. Chim. Acta 33, 1437 (1950) beschrieben. Nach diesem vorbekannten Verfahren wird das der obigen Formel II entsprechende Derivat, worin R jedoch Methyl bedeutet, d.h. (R)- oder (S)-1-[1-(4-Methoxybenzyl)-2-methyl-1,2,3,4,5,6,7,8-octahydro-isochinolin mit Phosphorsäure in 66%iger Ausbeute cyclisiert. Nachteilig an diesem Verfahren ist die niedrige Ausbeute in der Schlussstufe.

Aus Tetrahedron Letters (1987), 28, 4829 bzw. der Japanischen Patentpublikation 01034964 ist ein anderes Ausgangsmaterial, nämlich das der obigen Formel.II entsprechende Derivat, worin R jedoch Forrnyl bedeutet, d.h. (R)- oder (S)-1-[1-(4-Methoxybenzyl)-2-formyl-1,2,3,4,6,6,7,8-octahydroisochinolin, sowie dessen Ueberführung in Dextromethorphan bekannt, wobei allerdings keine Ausbeuteangaben vorhanden sind. Die Cyclisation des erwähnten 2-Formyl-octahydroisochinolinderivats zum Morphinangerüst mittels Polyphosphorsäure ist aus der FR-Patentschrift 1 584 396 bekannt. Jedoch ist diese Ueberführung nicht besonders attraktiv, muss doch das (R)-oder (S)-1-[1-(4-Methoxybenzyl)-2-formyl-1,2,3,4,5,6,7,8-octahydro-isochinolin entweder durch Racematspaltung (Ausbeute ≤ 50%) aus rac. 1-[1-(4-Methoxybenzyl)-1,2,3,4,5,6,7,8-octahydro-isochinolin und nachfolgende N-Formylierung oder gemäss den oben angegeben beiden Referenzen durch assymetrische Hydrierung aus dem nur aufwendig zugänglichen (Z)-1-[1-(4-Methoxybenzyliden)-1,2,3,4,5,6,7,8-octahydro-isochinolin-2-yl]methanon hergestellt werden. Mit der Entwicklung einer enantioselektiven Hydrierung von (Z)-1-[1-(4-Methoxybenzylgden)-1,2,3,4,5,6,7,8-octahydro-isochinolin-2-yl]-alkanonen wurde später ein einfacher Weg zur Herstellung von (R)- oder (S)-1-[1-(4-Methoxybenzyl)-1,2,3,4,5,6,7,8-octahydro-isochinolin-2-yl]alkanonen ohne Racematspaltung gefunden, vgl. z.B. die US Patentschrift 4,857,648.

Der vorliegenden Erfindung liegt deshalb die Aufgabe zugrunde, ein Verfahren zur Herstellung von (9a, 13a, 14a)-1-(3-Methoxymorphinan-17-yl)alkanonen der obigen Formel I durch Cyclisation eines (R)- oder (S)-1-[1-(4-Methoxybenzyl)-1,2,3,4,5,6,7,8-octahydro-isochinolin-2-yl]alkanons der obigen Formel II bereitzustellen, welches erlaubt, ein mittlerweile einfach zugängliches (R)- oder (S)-1-[1-(4-Methoxybenzyl)-1,2,3,4,5,6,7,8-octahydroisochinolin-2-yl]alkanon in guten Ausbeuten zu cyclisieren.

Im Rahmen der vorliegenden Erfindung wurde diese Aufgabe dadurch gelöst, dass die Cyclisation mittels einer Alkylsulfonsäure oder eines Gemisches von Alkansulfonsäuren unter den weiter unten im Detail beschriebenen ganz spezifischen Reaktionsbedingungen durchgeführt wird.

Demnach betrifft die vorliegende Erfindung ein Verfahren zur Herstellung von (9α, 13α, 14α)-1-(3-Methoxymorphinan-17-yl)alkanonen der obigen Formel I, welches dadurch gekennzeichnet ist, dass ein (R)- oder (S)-1-[1-(4-Methoxybenzyl)-1,2,3,4,5,6,7,8-octahydro-isochinolin-2-yl]alkanon der obigen Formel II mit einer Alkylsulfonsäure oder einem Gemisch von Alkylsulfonsäuren bei einer Temperatur zwischen 5° und 50°C, jedoch oberhalb des Schmelzpunktes der verwendeten Alkylsulfonsäure oder des verwendeten Alkylsulfonsäuregemisches, vorzugsweise zwischen 10°C und Raumtemperatur, cyclisiert wird.

Die Cyclisation eines (R)- oder (S)-1-[1-(4-Methoxybenzyl)-1,2,3,4,5,6,7,8-octahydro-isochinolin-2-yl]alkanons erfolgt in an sich bekannter Weise in der eingesetzten Säure, wobei diese gleichzeitig auch als Lösungsmittel dient. Für den Zweck der vorliegenden Erfindung eignen sich eine Alkylsulfonsäure oder ein Gemisch von Alkylsulfonsäuren, insbesondere Methan-, Ethan- und Propansulfonsäure, besonders bevorzugt Methansulfonsäure. Wenn die in Frage kommenden Alkylsulfonsäuren bei den bevorzugten Reaktionstemperaturen zwischen 10°C und Raumtemperatur fest sind und diese somit nicht gleichzeitig als Lösungsmittel dienen können, ist es notwendig, den Schmelzpunkt der Säure(n) durch Zugabe von bis zu 4%, vorzugsweise bis zu 2%, Wasser oder durch Zumischen einer anderen Alkylsulfonsäure soweit zu erniedrigen, dass die Reaktion oberhalb des Schmelzpunktes der Säure bzw. des Säuregemisches erfolgen kann. Bei Zugabe von >1% Wasser verringert sich die Umsetzungsschwindigkeit des Eduktes etwas, doch kann die Ausbeute bei entsprechend verlängerter Reaktionszeit konstant gehalten werden.

Das (R)- oder (S)-1-[1-(4-Methoxybenzyl)-1,2,3,4,5,6,7,8-octahydro-isochinolin-2-yl]alkanon wird vorteilhafterweise in geschmolzener Form (ca.85°C warm) zu der kalten Säure gegeben. Das (9α, 13α, 14α)-1-(3-Methoxylnorphinan-17-yl)alkanon wird entweder direkt aus der Säure durch Wasserzugabe bei 60°-65°C kristallisiert, oder nach Verdünnen mit Wasser mit einem unter den Reaktionsbedingungen inerten organischen Lösungsmittel, wie Toluol oder Methylenchlorid, vorzugsweise Toluol, extrahiert.

Die folgenden Beispiele zur Herstellung von (9α, 13α, 14α)-1-(3-Methoxymorphinan-17-yl)ethanon durch Cyclisation von (R)- oder (S)-1-[1-(4-Methoxybenzyl)-1,2,3,4,5,6,7,8-octahydro-isochinolin-2-yl]ethanon zeigen besonders vorteilhafte Ausführungsformen des erfindungsgemässen Verfahrens auf, sollen jedoch keinerlei Einschänkung darstellen. Alle Temperaturen sind in Celsiusgraden angegeben.

### Beispiel 1

In einem 750 ml Vierhalskolben mit Thermometer und Plattenrührer wurden unter einer Inertgasatmosphäre 200 g wasserfreie Methansulfonsäure vorgelegt und mit 2 ml Wasser versetzt. Zu dieser Lösung wurden innerhalb von 30 Minuten bei einer Temperatur von etwa 10-15° 30.40 g (100 mMol) etwa 85° warmes, geschmolzenes (R)- oder (S)-1-[1-(4-Methoxybenzyl)-1,2,3,4,5,6,7,8-octahydro-isochinolin-2-yl]ethanon (Gehalt: etwa 98%) in 4 Portionen gegeben. Das Reaktionsgemisch wurde im Wasserbad gerührt. Nach etwa 3 Stunden war das (R)- oder (S)-1-[1-(4-Methoxybenzyl)-1,2,3,4,-5,6,7,8-octahydro-isochinolin-2-yl]ethanon vollständig gelöst. Die braune Lösung wurde 6 Tage bei 10-21° gerührt. Der Reaktionsverlauf wurde gaschromatographisch überprüft

| Reaktionszeit [h] | Temperatur [°C] | Umsatz [%] |
|---|---|---|
| 0 | 10° | 0 |
| 24 | 12° | 25 |
| 48 | 14° | 55 |
| 72 | 17° | 78 |
| 96 | 20° | 89 |
| 120 | 21° | 98 |
| 144 | 21° | 99.5 |

### Beispiel 2

Zur braunen Lösung von Beispiel 1 wurden 400 ml Toluol und in einer Portion ein Gemisch von 150 g Eis und 330 mlWasser gegeben. Nach 5 Minuten Rühren wurde das Gemisch mit 2x 100 ml Toluol extrahiert. Die Toluolphasen wurden hintereinander mit 2x 100 ml W asser gewaschen. Die Wasserphasen wurden vereinigt und daraus die Methansulfonsäure zurückgewonnen. Die Toluolphasen wurden vereinigt und im Wasserstrahlvakuum eingedampft. Es wurden 30.25 g (92%) (9α, 13α,14α)-1-(3-Methoxymorphinan-17-yl)ethanon roh erhalten, Gehalt gemäss GC: 91%.

### Beispiel 3

Zur braunen Lösung von Beispiel 1 wurden bei 60° unter Rühren innerhalb von etwa 1 Stunde 600 ml Wasser getropft. Die erhaltene KristallSuspension wurde auf Raumtemperatur abgekühlt. Der Festkörper wurde abgenutscht, gut abgepresst und mit 100 ml Wasser nachgewaschen. Der Nutschkuchen wurde im Wasserstrahlvakuum 18 Stunden bei 35° getrocknet. Dabei wurden 27.81 g (88%) (9α, 13α, 14α)-1-(3-Methoxymorphinan-17-yl)ethanon erhalten, Gehalt gemäss GC: 95%.

## Patentansprüche

1. Verfahren zur Herstellung von (9α, 13α, 14α)-1-(3-Methoxymorphinan-17-yl)alkanonen der allgemeinen Formel 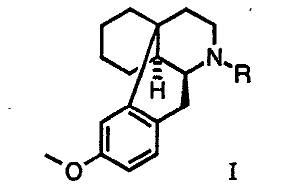 worin R C₂₄₋ Alkanoyl bedeutet,
**dadurch gekennzeichnet, dass** man ein (R)- oder (S)-1-[1-(4-Methoxybenzyl)-1,2,3,4,5,6,7,8-oetahydro-isochinolin-2-yl]alkanon der allgemeinen Formel 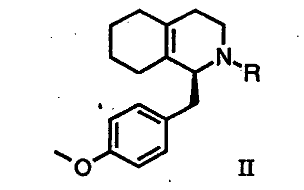 worin R die oben angegebene Bedeutung besitzt,
mit einer Alkylsulfonsäure oder einem Gemisch von Alkylsulfonsäuren bei einer Temperatur zwischen 5° und 50°C, jedoch oberhalb des Schmelzpunktes der verwendeten Alkylsulfonsäure oder des verwendeten Alkylsulfonsäuregemisches, cyclisiert.

2. Verfahren gemäss Anspruch 1, **dadurch gekennzeichnet, dass** Methan-, Ethan- oder Propansulfonsäure, vorzugsweise Methansulfonsäure, verwendet wird.

3. Verfahren gemäss Anspruch 1 oder 2, **dadurch gekennzeichnet, dass** die Cyclisation bei einer Temperatur zwischen 10°C und Raumtemperatur durchgeführt wird.

4. Verfahren gemäss einem der Ansprüche 1-3, **dadurch gekennzeichnet, dass** der Schmelzpunkt der Alkylsulfonsäure durch Zugabe von bis zu 4%, vorzugsweise bis zu 2%, Wasser oder durch Zumischen einer anderen Alkylsulfonsäure erniedrigt wird.

5. Verfahren gemäss einem der Ansprüche 1-4, **dadurch gekennzeichnet, dass** das (R)- oder (S)-1-[1-(4-Methoxybenzyl)-1,2,3,4,5,6,7,8-octahydroisochinolin-2-yl]alkanon in geschmolzener Form zu der kalten Säure gegeben wird.

6. Verfahren zur Herstellung von Dextromethorphan der Formel 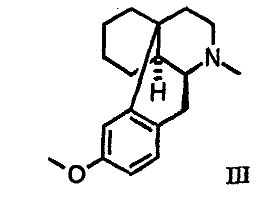 **dadurch gekennzeichnet, dass** man ein (R)- oder (S)-1-[1-(4-Methoxybenxyl)-1,2,3,4,5,6,7,8-octahydro-isochinolin-2-yl]alkanon der allgemeinen Formel 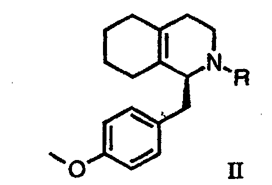 worin R die oben angegebene Bedeutung besitzt,
mit einer Alkylsulfonsäure oder einem Gemisch von Alkylsulfonsäuren bei einer Temperatur zwischen 5° und 50°C, jedoch oberhalb des Schmelzpunktes der verwendeten Alkylsulfonsäure oder des verwendeten Alkylsulfonsäuregemisches, cyclisiert und das erhaltene (9α, 13α, I4α)-1-(3-Methoxymorphinan-17-yl)alkanon der allgemeinen Formel 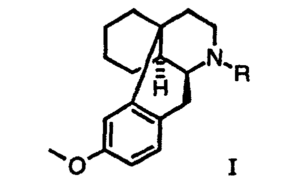 worin R die oben angegebene Bedeutung besitzt,
in an sich bekannter Weise durch Abspaltung des Restes R und N-Methylierung in Dextromethorphan überführt.

## Claims

1. A process for the production of (9α,13α,14α)-1-(3-methoxymorphinan-17-yl)alkanones of the general formula 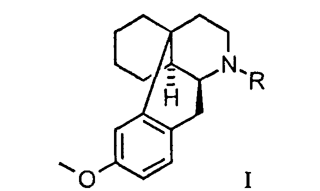 wherein R signifies C₂₋₄ - alkanoyl,
**characterized in that** a (R)- or (S)-1-[1-(4-methoxybenzyl)-1,2,3,4,5,6,7,8-octahydro-isoquinolin-2-yl]alkanone of the general formula 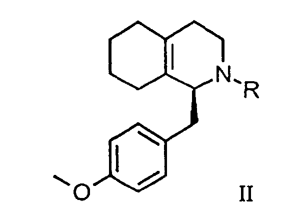 wherein R has the significance given above,
is cyclized with an alkylsulphonic acid or a mixture of alkylsulphonic acids at a temperature between 5°C and 50°C, but above the melting point of the alkylsulphonic acid used or of the alkylsulphonic acid mixture used.

2. A process according to claim 1, wherein methane-, ethane- or propanesulphonic acid, preferably methanesulphonic acid, is used.

3. A process according to claim 1 or 2, wherein the cyclization is carried out at a temperature between 10°C and room temperature.

4. A process according to any one of claims 1-3, wherein the melting point of the alkylsulphonic acid is lowered by the addition of up to 4%, preferably up to 2%, of water or by admixture of another alkylsulphonic acid.

5. A process according to any one of claims 1-4, wherein the (R)- or (S)-1-[1-(4-methoxybenzyl)-1,2,3,4,5,6,7,8-octahydro-isoquinoline is added in molten form to the cold acid.

6. A process for the manufacture of dextromethorphan of the formula 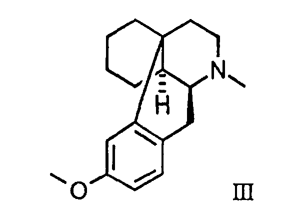 **characterized in that** a (R)- or (S)-1-[1-(4-methoxybenzyl)-1,2,3,4,5,6,7,8-octahydro-isoquinolin-2-yl]alkanone of the general formula 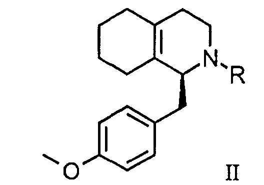 wherein R has the significance given above,
is cyclized with an alkylsulphonic acid or a mixture of alkylsuphonic acids at a temperature between 5°C and 50°C, but above the melting point of the alkylsulphonic acid used or of the alkylsuphonic acid mixture used, and the resulting (9α,13α,14α)-1-(3-methoxymorphinan-17-yl)alkanone of the general formula 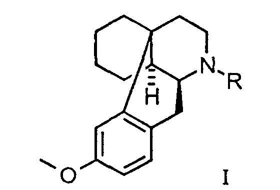 wherein R has the significance given above,
is converted into dextromethorphan in a manner known per se by cleavage of the residue R and N-methylation.

## Revendications

1. Procédé de préparation de (9α,13α,14α)-1-(3-méthoxymorphinan-17-yl)alcanones de formule générale 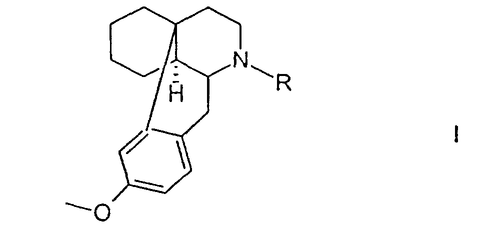 dans laquelle R est un radical alcanoyle en C₂₋₄,
**caractérisé en ce que** ce procédé comprend la réaction de cyclisation de la (R) ou (S)-1-[ 1-(4-méthoxybenzyl)-1,2,3,4,5,6,7,8-octahydro-isoquinoléin-2-yl]alcanone de formule générale 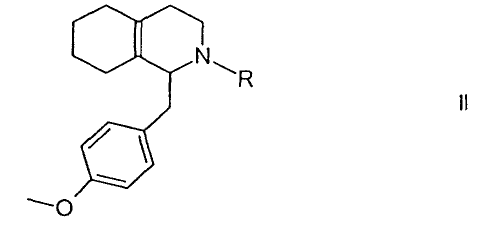 dans laquelle R est indiqué ci-dessus,
avec un acide alkyl sulfonique ou un mélange d'acides alkyl sulfoniques à une température comprise entre 5° et 50°C, toutefois au-dessus du point de fusion de l'acide alkyl sulfonique ou du mélange d'acides alkyl sulfoniques.

2. Procédé selon la revendication 1, **caractérisé en ce qu'**on utilise les acides méthyl, éthyl ou propyl sulfoniques et l'acide méthyl sulfonique de préférence.

3. Procédé selon les revendications 1 ou 2, **caractérisé en ce que** la cyclisation est effectuée à une température comprise entre 10°C et la température ambiante.

4. Procédé selon les revendications 1 à 3, **caractérisé en ce que** le point de fusion de l'acide alkyl sulfonique est abaissé par addition de jusqu'à 4 %, de préférence jusqu'à 2 % d'eau ou en ajoutant une autre acide alkyl sulfonique.

5. Procédé selon les revendications de 1 à 4, **caractérisé en ce que** la (R) ou (S)-1-[1-(4-méthoxybenzyl)-1,2,3,4,5,6,7,8-octahydro-isoquinoléin-2-yl] alcanone indiquée ci-dessus est ajoutée sous forme fondue à l'acide froid.

6. Procédé de préparation de dextrométhorphane de formule 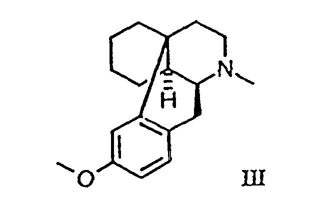 **caractérisé en ce qu'** il comprend la réaction de cyclisation de la (R) ou (S)-1-[1-(4-méthoxybenzyle)-1,2,3,4,5,6,7,8-octahydro-isoquinoléin-2-yl]alcanone de formule générale 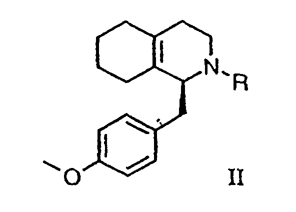 dans laquelle R est indiqué ci-dessus,
avec un acide alkyl sulfonique ou un mélange d'acides alkyl sulfoniques à une température comprise entre 5° et 50°C, toutefois au-dessus du point de fusion de l'acide alkyl sulfonique ou d'un mélange d'acides alkyl sulfoniques, et que le (9α,13α,14α)-1-(3-méthoxymorphinan-17-yl)alcanone de formule générale 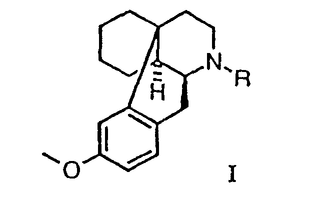 dans laquelle R est indiqué ci-dessus,
obtenue dans cette étape est convertie en dextrométhorphane en utilisant des procédés bien connus comme les réactions d'élimination du reste R et de la méthylation de N.
